# EUROPEAN PATENT APPLICATION

(11) **EP 3 412 995 A2**
(43) Date of publication of application: **12.12.2018**
(21) Application number: 18174301.4
(22) Date of filing: 25.05.2018
(51) Int. Cl.: F25D 3/08, B65D 21/02, A61F 7/10

(54) **STACKABLE FREEZER PACK**

(30) Priority: 09.06.2017 US 201715731449
(71) Applicant: Weber, Heinz, Beaconsfield, Quebec H9W 5H4 (CA)
(72) Inventor: Weber, Heinz, Beaconsfield, Quebec H9W 5H4 (CA)
(74) Representative: Glück Kritzenberger Patentanwälte PartGmbB

(57) **Abstract**

A freezer pack which has a body (10) in the shape of a cuboid, a first face (14) of the body having a plurality of recesses (26) and a second face (12) opposite to the first face having a plurality of projections (24) extending outwardly therefrom, the projections and the recesses being sized and located such that a projection on the second face will mate with a recess on a first face of a second body in the shape of a cuboid.

## Description

### FIELD OF THE INVENTION

The present invention relates to freezer packs and more particularly, relates to an improved freezer pack and a freezer pack system.

### BACKGROUND OF THE INVENTION

The use of freezer packs among consumers is widespread. The freezer packs are normally designed so as to have a body with a coolant placed therein. The freezer pack is utilized to keep objects cooler than would normally be the case. Typically, when it is desired to transport food from a first location to a further location, and the food is either cold or frozen, freezer packs are placed next to the food to retard any warming of the food. Although reference is made herein to food, the freezer pack may obviously be utilized for other similar purposes.

Generally, the freezer packs are in the form of a container which holds the coolant therein. The container may either be of a rigid material or alternatively, may be formed of a flexible material. Often, freezer packs formed of a flexible material are utilized to cool down body parts, which is frequently required in the case of an injury such as a sprain or the like. However, the flexible or rigid containers may be utilized interchangeably.

### SUMMARY OF THE INVENTION

It is an object of the present invention to provide an improved freezer pack which is stackable and can provide greater cooling efficiency.

The present invention is directed to a freezer pack comprising a body in the shape of a cuboid, a first face of the body having a plurality of recesses, a second face of the body opposite to the first face having a plurality of projections extending outwardly therefrom, the projections and the recesses being sized and located such that a projection on the second face will mate with a recess on a first face of a second body in the shape of a cuboid.

The present invention also provides a freezer pack system comprising first and second freezer packs, each freezer pack having a body in the shape of a cuboid, a first face of each of the bodies having a plurality of recesses, a second face opposite to the first face having a plurality of projections extending outwardly therefrom, the projections on the first body being sized and located to fit within the recesses on the first face of the second body.

The term "freezer pack" is used herein for any container which is designed to be utilized to cool (and sometimes warm) an adjacent object. These freezer packs are widely available and are also known as gel packs, ice packs, cold packs etc.

The freezer packs or gel packs contain a coolant which may be selected from known materials widely used for such a purpose. Generally, water, a refrigerant gel or a like fluid is utilized. The coolant can absorb a considerable amount of heat due to the high latent heat of fusion of water. When using water, additives to improve the properties thereof are often used - i.e. substances to prevent bacterial growth in the pack and materials to prevent the water from solidifying so that it remains a thick gel throughout use. Gel packs have been known to be made by adding hydroxy ethyl cellulose, sodium polyacrylate, vinyl-coated silicone gel, etc.

As mentioned above, the ice packs are occasionally used as a means of warming an item rather than cooling. Both uses fall within the scope of the present invention.

In the preferred embodiment of the present invention, the package is formed of a suitable plastic material such as polyethelene. Many plastic materials are widely known in the art and one skilled in the art can readily select a suitable material.

The body of the freezer pack has a generally cuboid configuration. It will be understood that although the configuration is "substantially" or "generally" cuboid, it may differ from a true cuboid configuration by having, for example, rounded corners and the like. However, the freezer pack does have six faces with two of the faces being generally recognized as an upper face and a bottom face with the remaining four faces being end and/or side walls. For purposes of this application, the term "cuboid" will include such deviations from a true cuboid recognizing that the key elements would be the six faces of the package.

According to the present invention, the upper face will have a plurality of recesses formed therein. Preferably, the number of recesses is a multiple of four with at least one of each of the recesses being located proximate a corner of the upper face. Preferably the number of recesses/projections will be any of four, eight or sixteen.

On the bottom face, the cuboid will be provided with a plurality of projections, each of the projections being sized and arranged to mate with a recess of an adjoining first face of a further cuboid. The use of projections and recesses for locking materials together is well known in the art and need not be elaborated on herein. It suffices to say that preferably the projections and recesses are designed and arranged so as to have a "snap fit" arrangement wherein one can sense and hear that the projections and recesses fit together. Such an arrangement is well known in the art and widely used, for example, in the case of "toy" building blocks.

Utilizing the arrangement of the present invention, it has been found that not only does one provide a stackable freezer pack which can more easily be stored in a freezer or the like, but the freezer pack also has a higher efficiency in heat transfer. In some instances, the increased efficiency can amount up to approximately 30%. This can be the result of increased surface area of the freezer pack of the present invention.

In a preferred embodiment of the present invention, there are provided three different sizes of freezer packs - a first one having a somewhat square top and bottom face with four recesses and projections thereon. A second size would be rectangular and have eight projections and recesses in a four by two configuration while a still larger size would be substantially square and have sixteen such recesses and projections arranged in a four by four configuration.

In one embodiment of the present invention, one of the faces will have "channels" formed therein. These channels will not only serve to increase the surface area and thus the thermal efficiency of the package, but they may also serve as a reservoir for condensation formed on the package. Preferably, the channels will be formed on the face which has recesses and the recesses will collect any moisture present. Thus, if desired, the channels may be angled so as to direct the moisture to the recesses.

The ice pack of the present invention may also have anti-expansion members. Thus, when the relatively larger top and bottom faces are utilized, the top and bottom faces may have a member connecting the two faces so as to limit expansion of the body. The provision of such anti-expansion members will also again increase the surface area of the ice pack which will result in a greater thermal efficiency.

### BRIEF DESCRIPTION OF THE DRAWINGS

Having thus generally described the invention, reference will be made to the accompanying drawings illustrating embodiments thereof, in which:
Figure 1 is a perspective view of an ice pack container according to one embodiment of the present invention;
Figure 1A is a cross-sectional view taken along the line A-A of Figure 1.

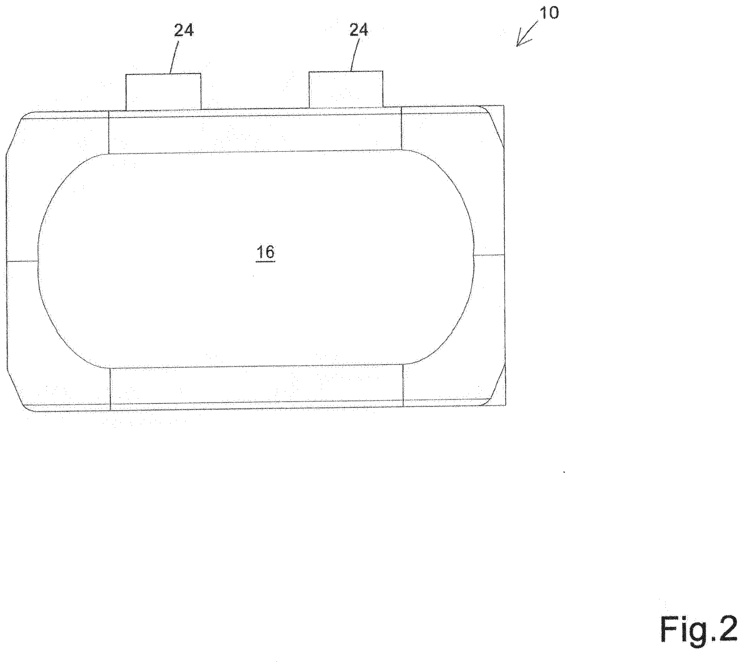

Figure 2 is a side elevational view thereof;

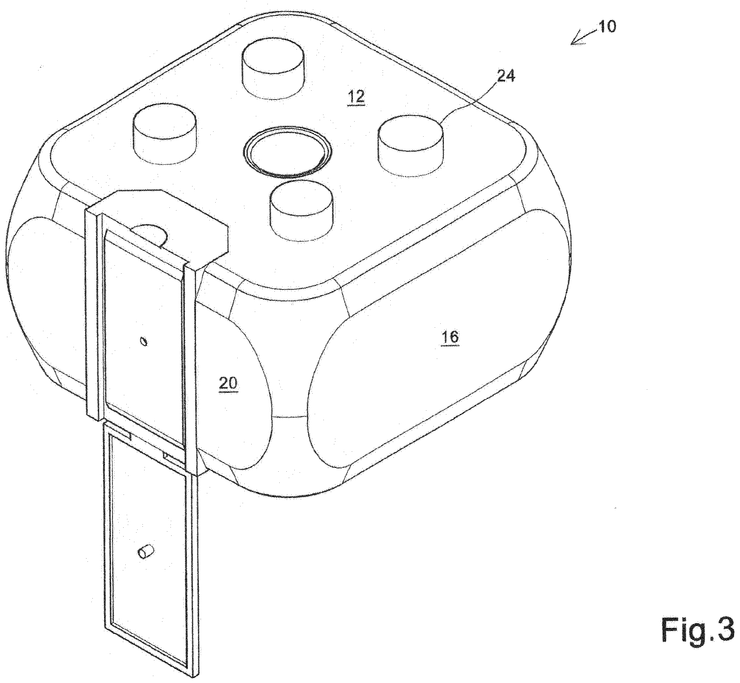

Figure 3 is a perspective view of the ice pack container of Figure 1 showing a sealing member in a partially open position;

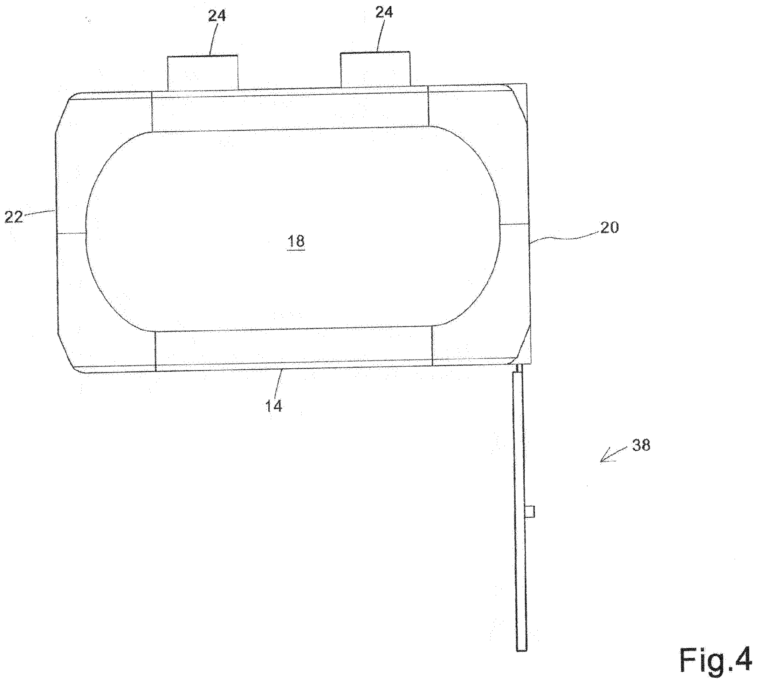

Figure 4 is an elevational view as seen from the left hand side of Figure 3;

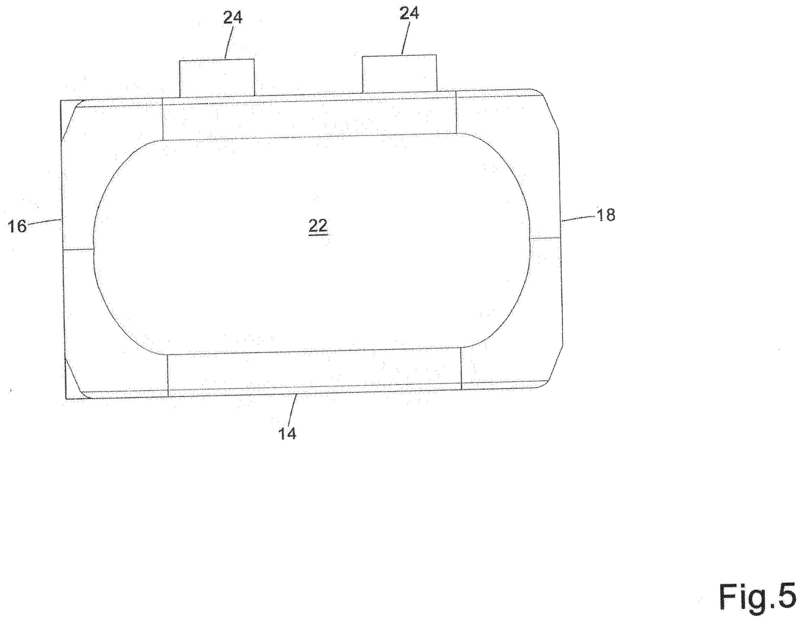

Figure 5 is an end elevational view thereof;

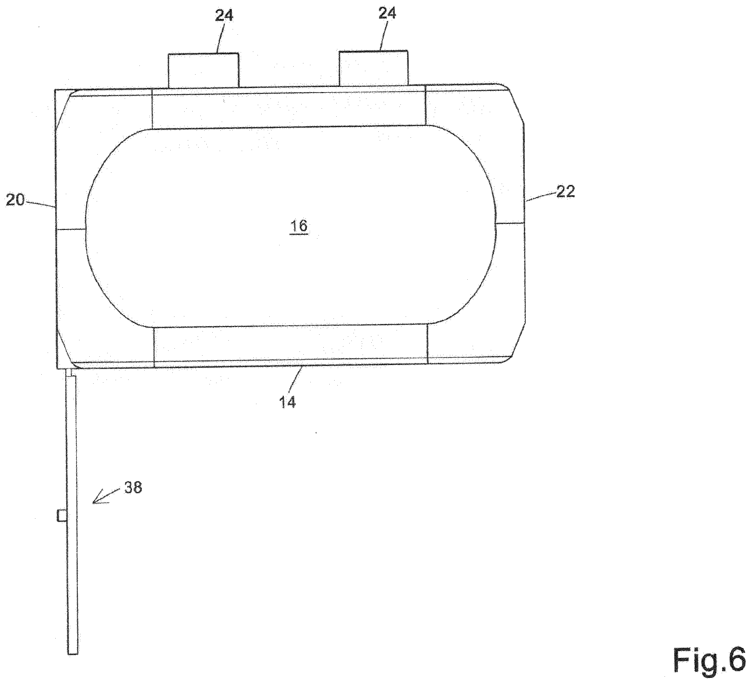

Figure 6 is a side elevational view thereof;

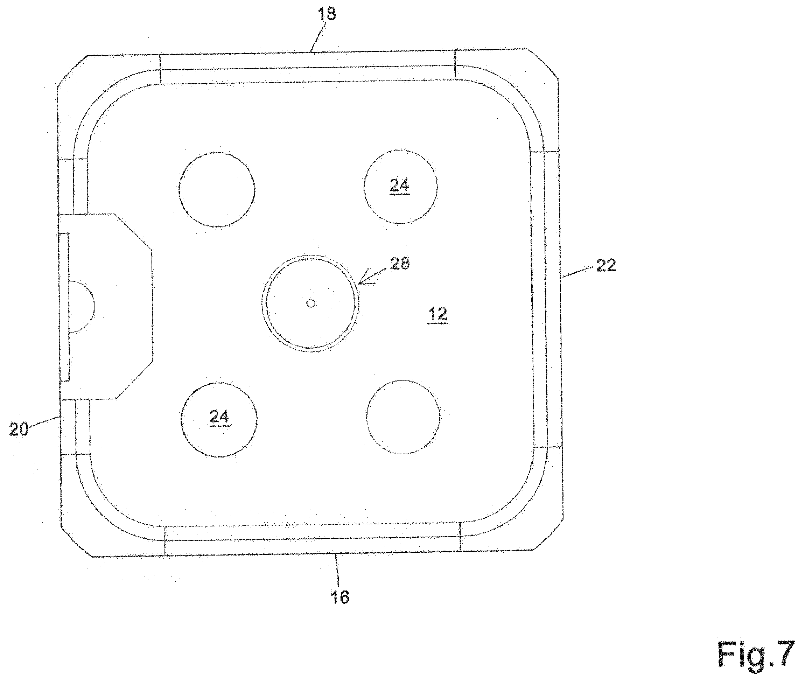

Figure 7 is a top plan view thereof;
Figure 8 is a bottom plan view thereof;
Figure 9 is a perspective view of a further embodiment of an ice pack according to the present invention;

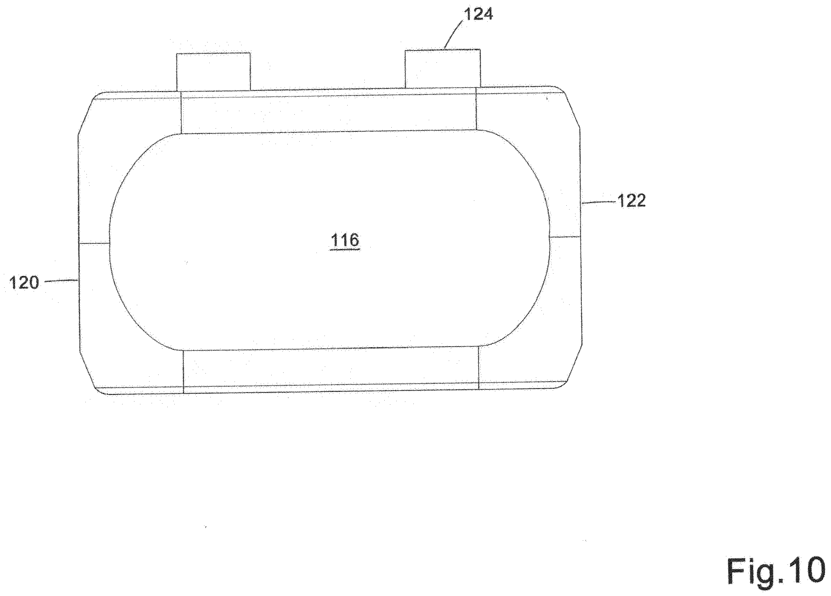

Figure 10 is an end elevational view thereof;

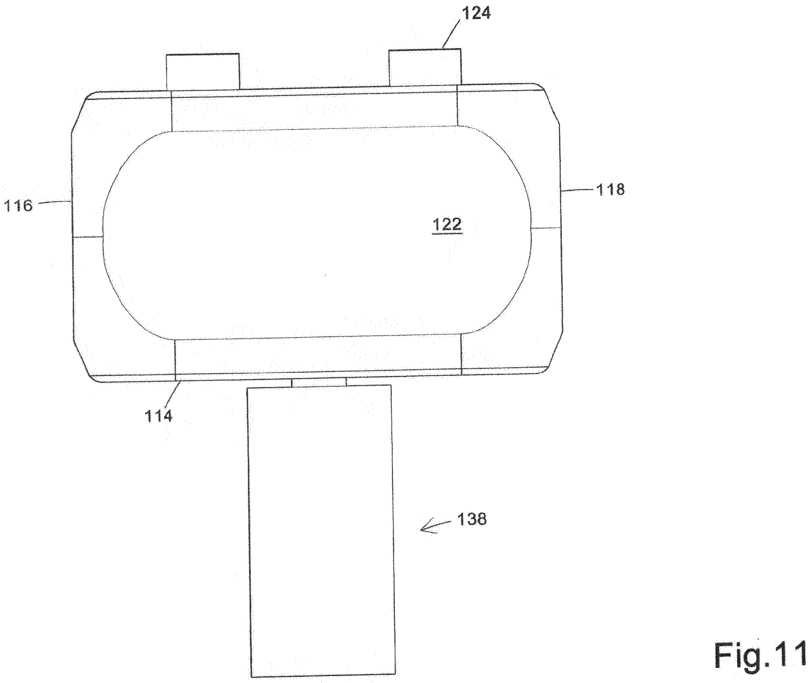

Figure 11 is a side elevational view thereof;

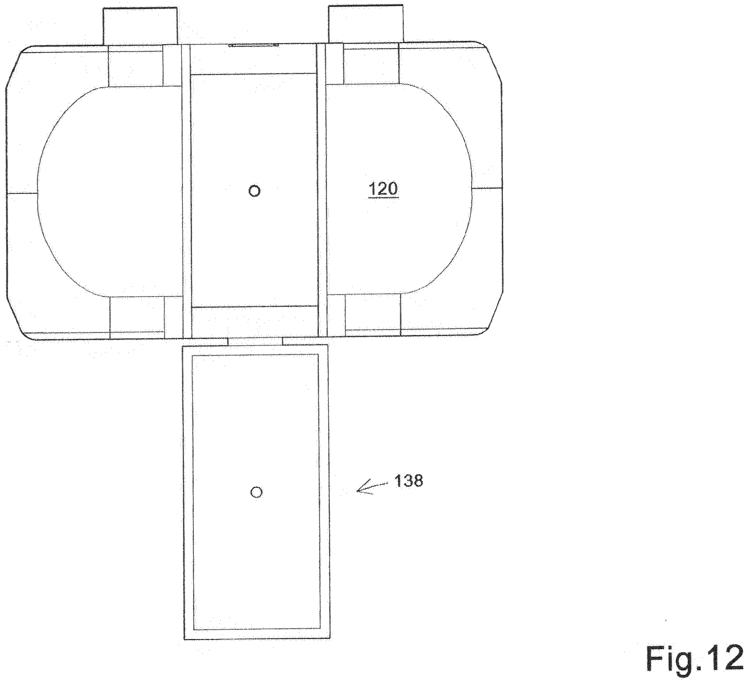

Figure 12 is an end elevational view with the cover being in an open position;

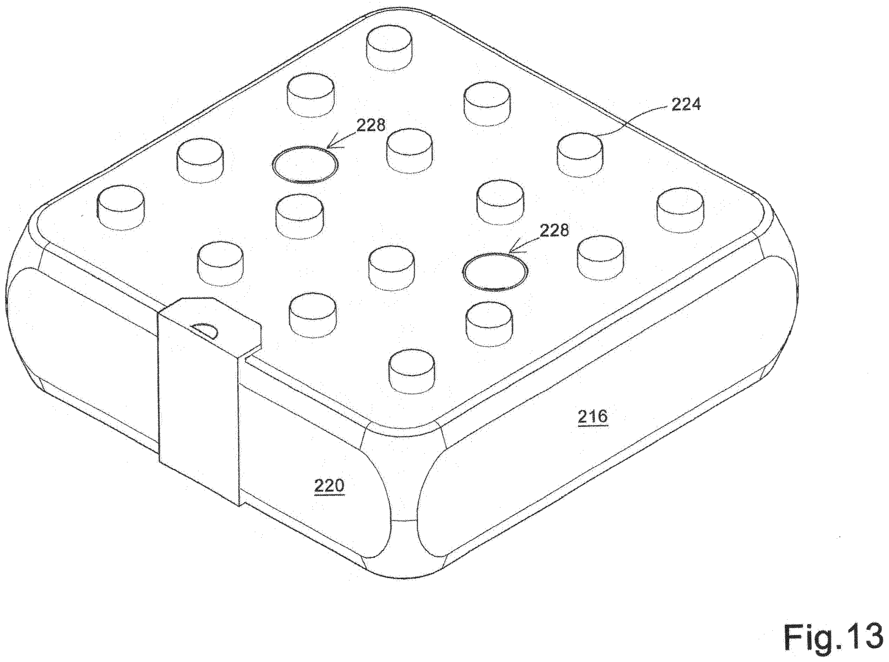

Figure 13 is a perspective view of a further embodiment of the present invention;

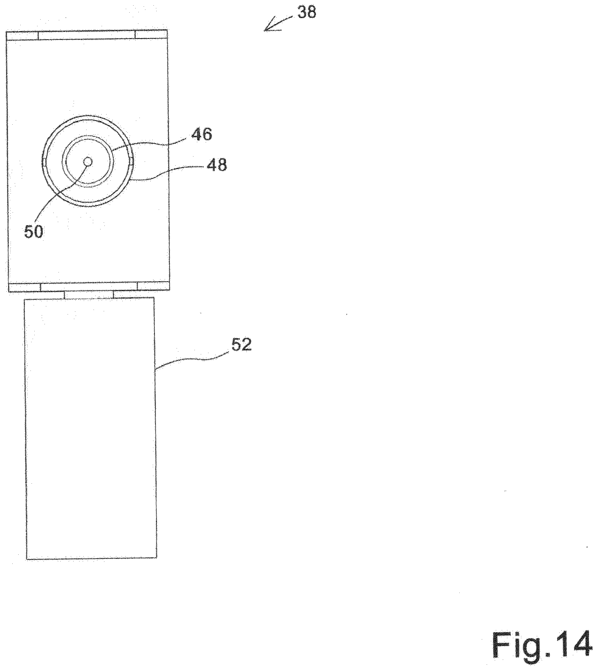

Figure 14 is a front view of a sealing member according to the present invention;

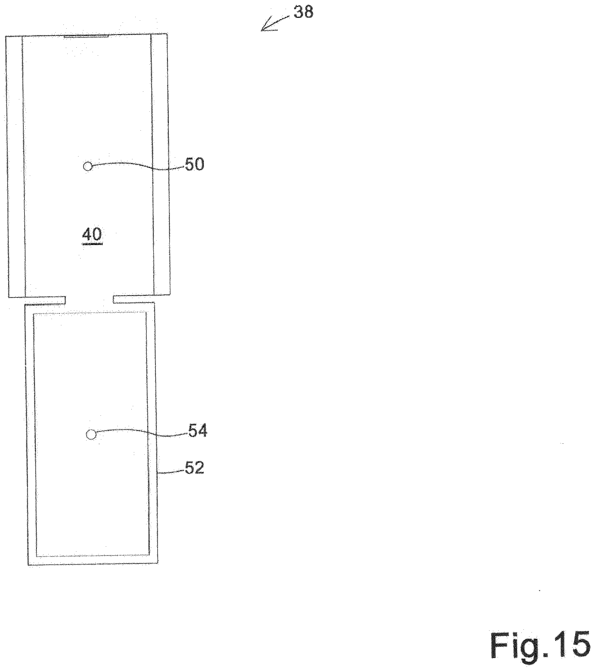

Figure 15 is a rear view thereof;

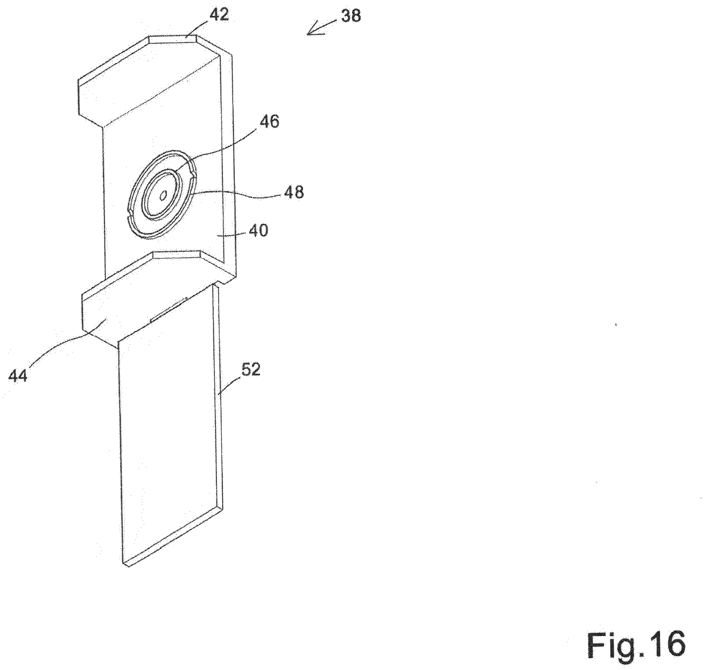

Figure 16 is a perspective view of a sealing member in an open position;

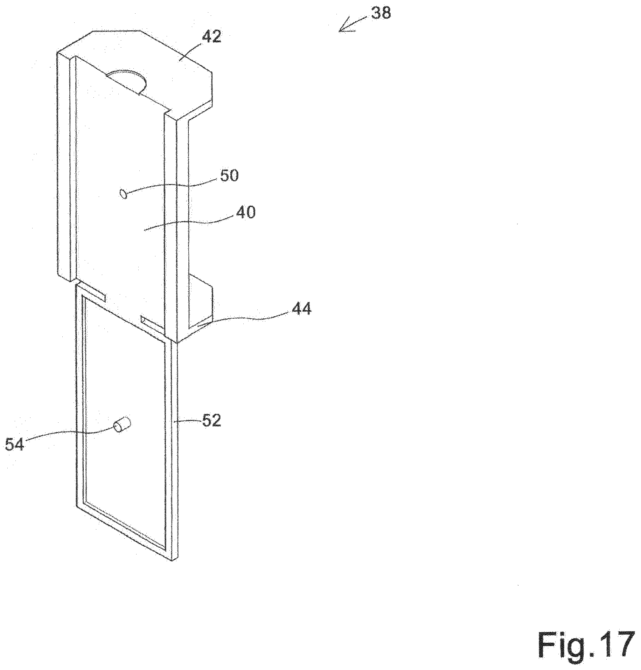

Figure 17 is a perspective view thereof;

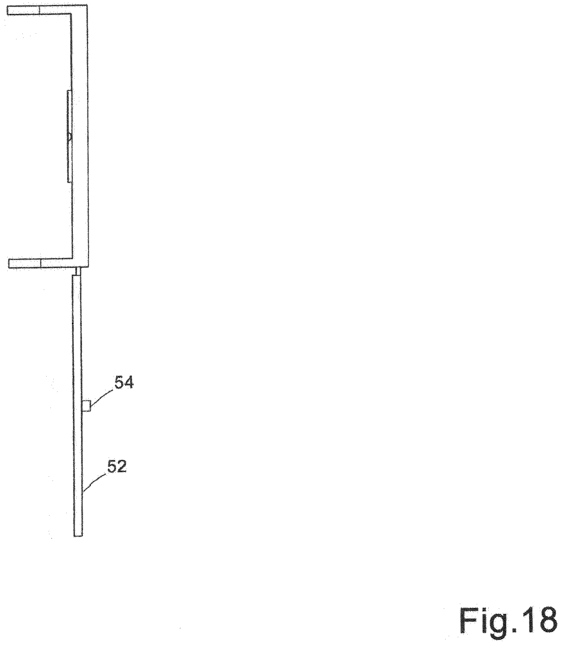

Figure 18 is a side elevational view thereof;

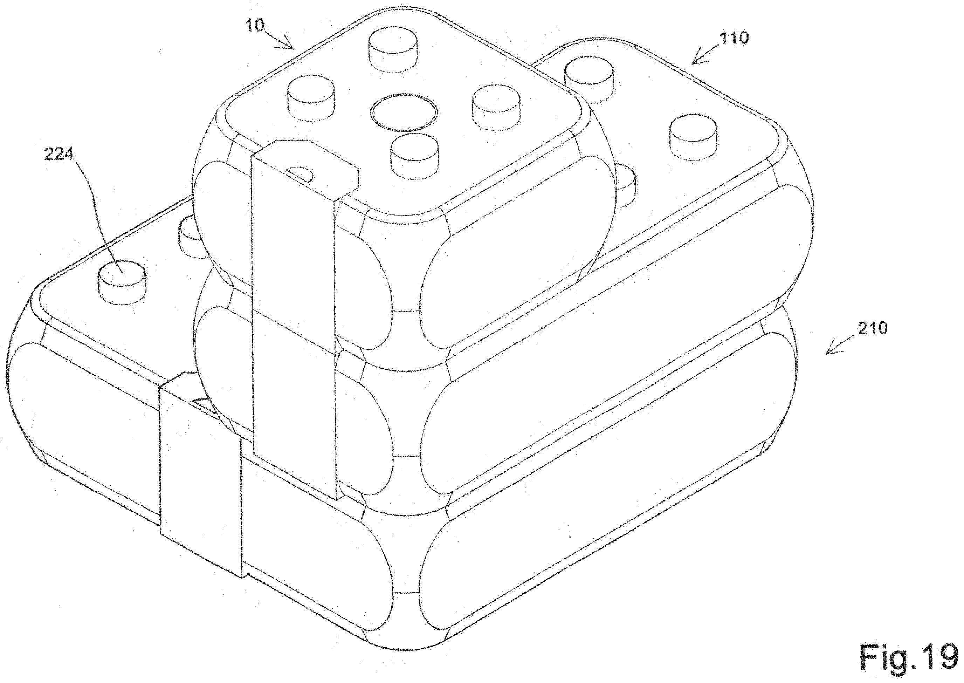

Figure 19 is a perspective view showing the stackability of the ice pack units according to the present invention; and

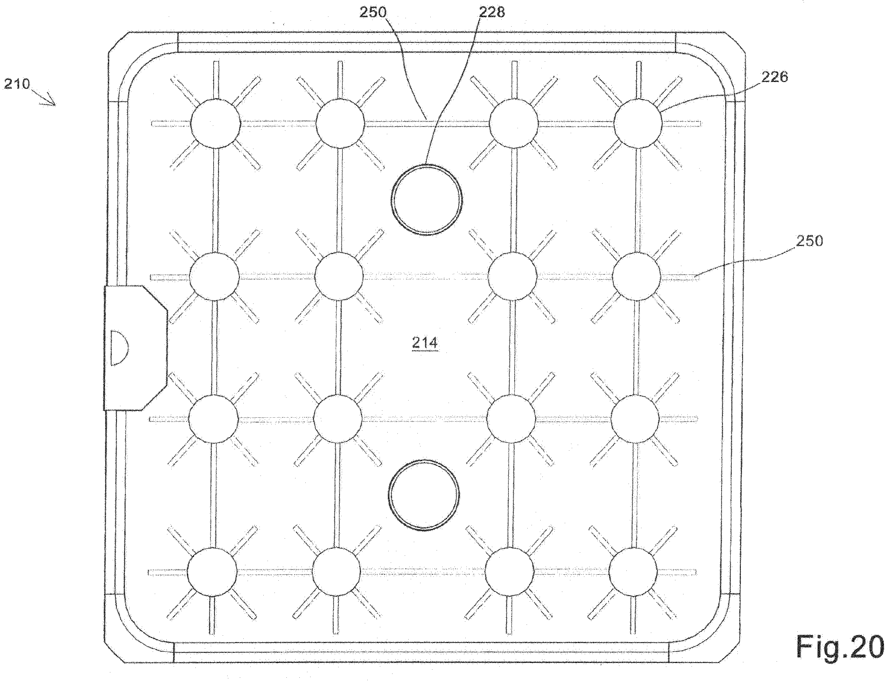

Figure 20 is a bottom view of a further embodiment of the present invention.

### DETAILED DESCRIPTION OF THE INVENTION

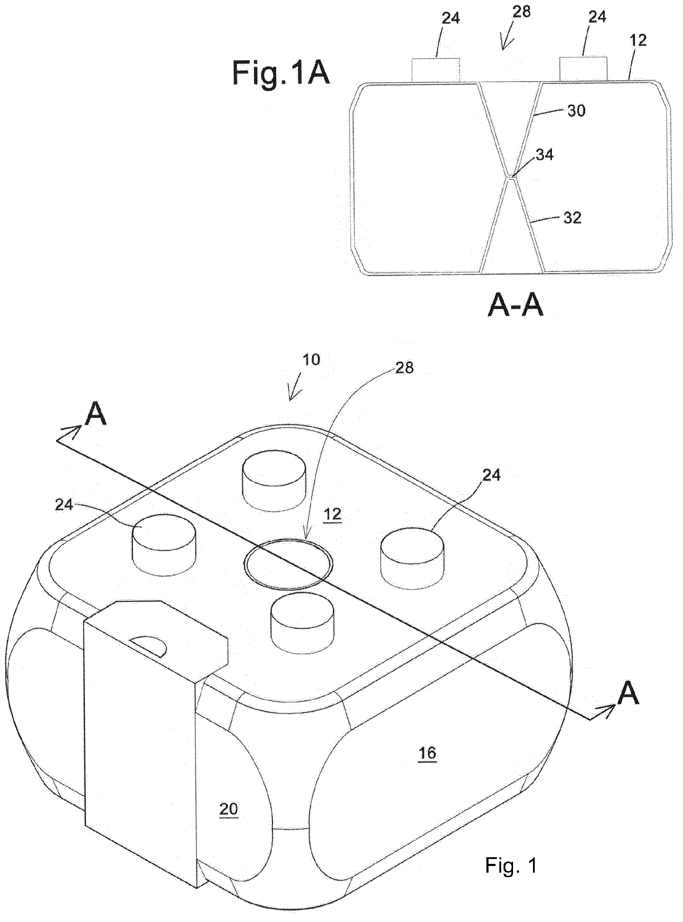

Referring to the drawings in greater detail and by reference characters thereto, there is illustrated in Figure 1 a first embodiment according to the present invention and which ice pack unit is generally referred to by reference numeral 10.

Ice pack 10 is in the general form of a cuboid. While the general configuration is cuboidal, the ice pack will have rounded corners. Ice pack 10 includes a top wall 12, and a bottom wall 14. Ice pack 10 also includes a pair of side walls 16, 18 and a pair of end walls 20, 22.

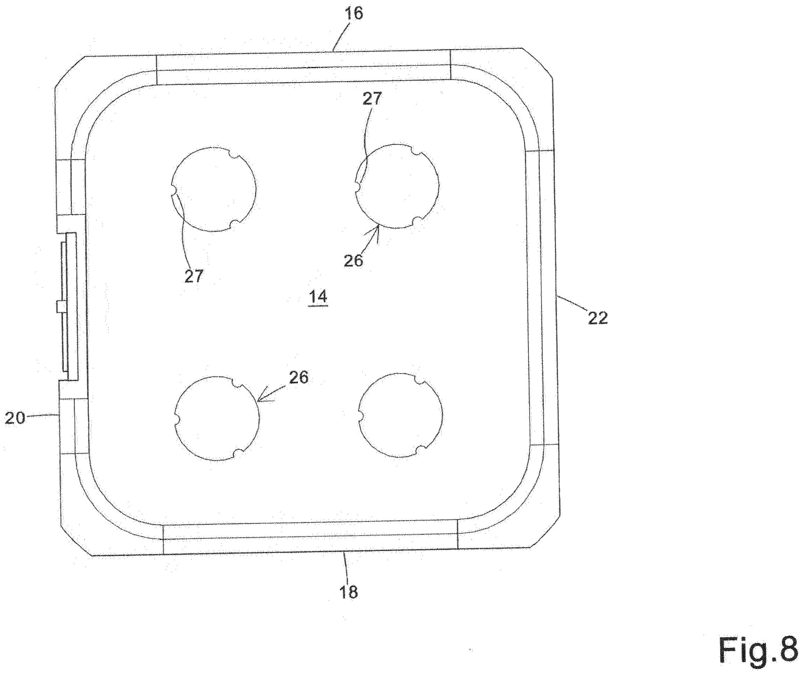

Ice pack 10 has, on its top wall 12, a plurality of projections 24 while on a bottom wall 14, there are provided a plurality of recesses 26. Each of projections 24 and recesses 26 is sized such that a projection 24 will engage with a recess 26 preferably in a snap fit relationship. Thus, as may be seen in Figure 8, the interior wall of surrounding recess 26 may include protrusions 27 which will act to provide a tight seal with projections 24. Alternatively, the wall recess 26 may be provided with an undercut and projections 24 may have a slightly enlarged end. Such an arrangement is well known in the art. It suffices to say that a person skilled in the art is able to provide a snap fit arrangement.

An anti-expansion member 28, in the illustrated embodiment, comprises an inwardly extending conical member 30 which is formed on top wall 12 and a inwardly extending conical member 32 formed in bottom wall 14. Conical members 30 and 32 meet to have a common wall 34. This arrangement limits the expansion of ice pack 10.

In an end wall 20 there is provided a filling aperture 36 which will have a raised collar or flange 37 extending thereabout.

A sealing member 38 generally designated by reference numeral 38 has a central wall 40 and a pair of end walls 42 and 44. Central wall 40 has an inner circular flange 46 and an outer circular flange 48 extending outwardly therefrom. Flanges 46 and 48 are designed to fit around the raised collar or flange 37 to thereby seal the filling aperture 36. Central wall 40 is also provided with a relatively small aperture 50 formed therein. A lid or cover 52 is hingedly connected to sealing member 38 and on one surface thereof, has a projection 54 which is designed to engage with aperture 50 to retain lid 52 in position.

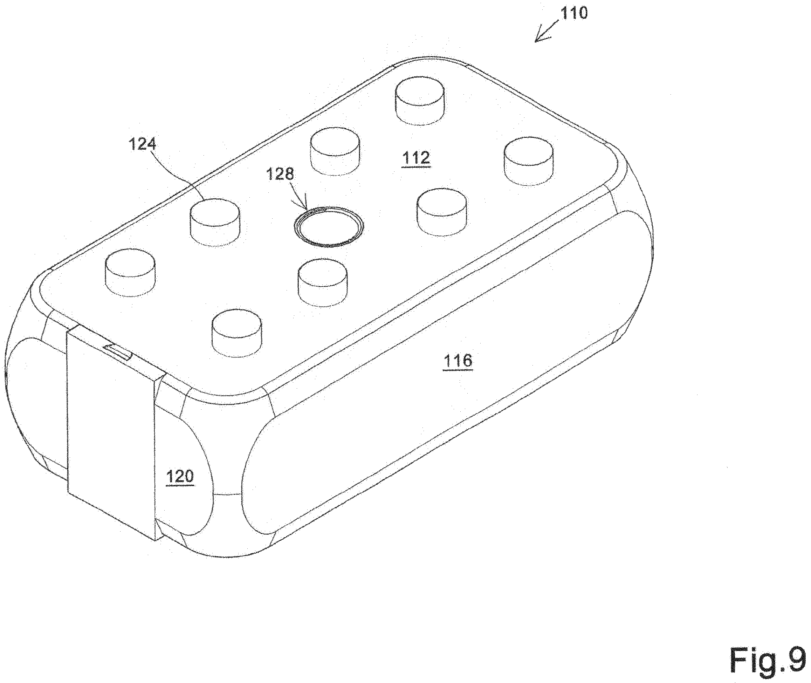

A larger ice pack unit 110 is illustrated in Figure 9. Ice pack 110 has many common features with ice pack 10 and similar reference numerals in the 100's are employed for similar components. Thus, ice pack unit 110 will have a configuration in the form of a cuboid. Ice pack 110 includes a top wall 112 and a bottom wall 114. Ice pack unit 110 also includes side walls 116, 118 and end walls 120, 122. On top wall 112 there are provided a plurality of projections 124 with corresponding recesses being provided on bottom wall 114. In the illustrated arrangement, there are eight such projections with the ice pack unit 110 having a length double that of ice pack 10.

A further embodiment of the invention includes an ice pack 210 which also has many common features with ice pack 10 and similar reference numerals in the 200's are employed for similar components. Ice pack 210 also has a cuboid configuration with substantially square top wall and bottom wall. In the illustrated embodiment, it will be seen that there are a total of sixteen projections and recesses in a four by four configuration. In this embodiment, on bottom wall or face 214, there are provided a plurality of channels 250 which are in communication with recesses 226. Channels 250 are arranged to direct any condensation or the like to the recesses 226. Also, as previously mentioned, they provide an increased surface area and an increased thermal efficiency. Channels 250 may, if so desired, have a slope to direct moisture to the recesses 226.

It will be understood that the above described embodiments are for purposes of illustration only and that changes and modifications may be made thereto without departing from the spirit and scope of the invention.

## Claims

1. A freezer pack comprising:
a body in the shape of a cuboid;
a first face of said body having a plurality of recesses;
a second face of said body opposite to said first face having a plurality of projections extending outwardly therefrom;
said projections and said recesses being sized and located such that a projection on said second face will mate with a recess on a first face of a second body in the shape of a cuboid.

2. The freezer pack according to Claim 1 wherein said body is formed of a plastic material.

3. The freezer pack according to Claim 1 or Claim 2 wherein said body is filled with a coolant.

4. The freezer pack according to any of Claims 1 to 3 wherein said first face of said body has a plurality of recesses, said plurality of recesses being a multiple of four.

5. The freezer pack according to any of Claims 1 to 4 wherein said first face and said second face have an anti expansion member securing said first and second faces together.

6. The freezer pack according to any of Claims 1 to 5 wherein one face of said cuboid has an aperture formed therein, a plug sealing said aperture, and a cover meeting with said cuboid to limit access to said plug and aperture.

7. The freezer pack according to any of Claims 1 to 6 wherein said first face of said body has a plurality of channels communicating with at least one of said recesses.

8. A freezer pack system comprising first and second freezer packs according to any of Claims 1 to 7.
